# EUROPEAN PATENT SPECIFICATION

(11) **EP 1 593 381 B1**
(45) Date of publication and mention of the grant of the patent: **07.11.2007**
(21) Application number: 04380229.7
(22) Date of filing: 17.11.2004
(51) Int. Cl.: A61K 33/30, A61P 1/12

(54) **Zinc oxide based formulation for preventing and treating diarrhea in farm animals**
Zinkoxid enthaltende Zusammensetzungen zur Behandlung oder Vorbeugung von Diarrhöe bei Tieren
Compositions a base d'oxyde de zinc pour le traitement et la prevention de la diarrhee chez l'animal

(30) Priority: 26.11.2003 ES 200302779
(43) Date of publication of application: 09.11.2005
(73) Proprietor: Tecnologia & Vitaminas, S.L., 43006 Tarragona (ES)
(72) Inventor: Simo Grifoll, Jordi, 43365 Alforja Tarragona (ES)
(74) Representative: Carvajal y Urquijo, Isabel

(56) References cited:
- EP-A- 0 414 605
- WO-A-93/14773
- WO-A-97/17406
- WO-A-20/04080210
- CH-A- 684 387
- POULSEN HANNE DAMGAARD: "Zinc oxide for weanling piglets" ACTA AGRICULTURAE SCANDINAVICA SECTION A ANIMAL SCIENCE, vol. 45, no. 3, 1995, pages 159-167, XP008049973 ISSN: 0906-4702
- JENSEN-WAERN MARIANNE ET AL: "Dietary zinc oxide in weaned pigs: Effects on performance, tissue concentrations, morphology, neutrophil functions and faecal microflora" RESEARCH IN VETERINARY SCIENCE, vol. 64, no. 3, May 1998 (1998-05), pages 225-231, XP002336743 ISSN: 0034-5288
- DATABASE BIOSIS [Online] BIOSCIENCES INFORMATION SERVICE, PHILADELPHIA, PA, US; 1998, PEJSAK Z ET AL: "Effectiveness of zinc oxide in prophylaxy of post-weaning diarrhoea of pigs" XP002337928 Database accession no. PREV199900461097 & POLISH JOURNAL OF VETERINARY SCIENCES, vol. 1, no. 2, 1998, pages 9-13, ISSN: 1505-1773

## Description

### FIELD OF THE INVENTION

The present invention refers to a new zinc oxide based formulation, in liquid form, for preventing and treating diarrhea in farm animals.

### STATE OF THE ART

It is known in the state of the art that the administration of zinc oxide in pig and goat food for the first 21 days or between days 14 and days 42, according to sources, achieves positive effects in the development of these animals. Weight gain and prevention of diarrhea due to E. Coli stand out among these positive effects.

These effects are disclosed in a number of works which can be found in the literature, and the following works can be listed, without being limited thereto:
⇒ Pejsak, Z.; Markowska-Daniel, I.; Mokrzycka, A.; Szkoda, J.; Rutkowska-Pejsak, B.; "Effectiveness of zinc oxide in prophylaxy of post-weaning diarrhea of pigs", Polish Journal of Veterinary Sciences (1998), 1(2), 9-13.
⇒ Xu, Zirong; Wang, Minqi; "Effect of zinc oxide and zinc proteinate on growth and digestion of weaning pigs"; Zhejiang Nongye Daxue Xuebao (1999), 25(1), 103-106.
⇒ Monteiro De Lima, Gustavo Julio Mello; Mores, Nelson; Fialho, Flavio Bello; Paiva e Brito, Maria Aparecida Vasconcelos; Gomes, Paulo Cezar; "Effects of the period of zinc supplementation in the diet on the performance of weaned pigs"; Revista da Sociedade Brasileira de Zootecnia (1994), 23(6), 949-58.
⇒ Bertol, Teresinha Marisa; Guimarues de Brito, Benito, "Effect of zinc oxide and copper sulfate with and without feed restriction on post-weaning performance and occurrence of diarrhea in piglefs"; Revista da Sociedade Brasileira de Zootecnia (1995), 24(2), 278-88.
⇒ Poulsen, Hanne Damgaard; "Zinc oxide for weaning piglets"; Acta Agriculturae Scandinavica, Section A: Animal Science (1995), 45(3), 159-67.
⇒ Smith, B.L.; Embling, P.P.; "The influence of chemical form of zinc on the effects of toxic intraruminal doses of zinc to sheep"; Journal of Applied Technology (1984), 4 (2), 92-6.

According to all these documents, zinc oxide is administered in solid state as a direct additive in the food or water administered to the animals. Zinc oxide is a commercial product, usual on the chemical market, such that there is the risk that farmers use products of low quality and little purity, due to their lack of knowledge or to save costs, which in many cases are not suitable for animal and/or human consumption, even being able to be contaminated with prohibited heavy metals, unsuitable organic residues, etc. Since It is difficult to control the quality in the administration of zinc oxide in the livestock industry, it could therefore possibly lead to producing animals with residues of prohibited products and which are therefore harmful to man.

On the other hand, the administration of zinc oxide in solid state, added to the food or water for animals, normally causes rejection by the animal due to the flavor, appearance, hardness of the solid, etc.

As a result of the foregoing, there was a need to provide an alternative manner of administering zinc oxide to animals, due to the excellent results the administration thereof produces in the animal diet, which would in turn enable an administration with quality assurances and without risks of rejection by the animals.

### DESCRIPTION OF THE INVENTION

It has now been found that the administration of zinc oxide, in the form of a liquid formulation containing the zinc oxide in suspension, provides an administration form accepted by the animals, and which in turn ensures the purity and non-contamination necessary for administration in animals.

This formulation comprises a zinc oxide suspension at a high concentration in water, together with surfactants facilitating the formation of said suspension.

Thus, according to a first aspect, the present invention refers to a zinc oxide based formulation for preventing and treating diarrhea in farm animals, comprising:
from 30 to 60% by weight of zinc oxide, with a mean particle size of less than 20 microns,
from 1 to 6% of a nonionic surfactant,
from 2.5% to 10% of an ionic surfactant, and
water up to 100% of the volume, all the percentages being by weight.

The formulation has been developed to be applied in farm animal, ; its use has been shown to be especially effective in pigs and rabbits.

Preferably, the nonionic surfactant is a tristirylphenol ethoxylate or an ethoxylated ester of phosphoric acid, and the ionic surfactant is calcium dodecylbenzene sulfonate or calcium phenyl sulfonate. These surfactants have the main advantage of not causing rejection due to a bitter and unsuitable flavor, and they are acceptable for animal consumption.

According to a preferred embodiment, other components can be added to the formulation to enhance the indicated effect, for example, one or more flavoring agents, flavor enhancers, proteins, vitamins, antibiotics and other drugs.

According to a second aspect, the present invention refers to the use of a zinc oxide based liquid formulation according to that described above, in the preparation of a medicament for preventing and treating diarrhea in farm animals, and especially in pigs and rabbits.

It has been observed that this formulation is not rejected by the animals ingesting it. Furthermore, improved effects of weight gain, food consumption, serum protein increase and as a preventive agent of diarrhea in animals to which it is administered, are achieved. Furthermore, the formulation is perfectly stabilized with regard to pH, particle size and components, and it is easy to handle, given that its dosing can be carried out by volume and by weight, whereby it can be adapted to any dosing and/or administration system found in the area of the mixture.

Since it is a liquid and stabilized product, it can also be administered in drinking water, since the mixture would remain in suspension for at least several hours due to its small particle size, whereby a new administration form is achieved, since the precipitations which occur when zinc oxide is used directly are prevented, in addition to the drawbacks of wettability occurring with its use.

The invention will be furthermore described below, by way of illustration and by no means limiting, by means of the following examples:

### EXAMPLES

### Example 1: Suspension with 50% w/v of zinc metal

800 liters of water, 20 Kg of Soitem 132 SP® (tristirylphenol ethoxylate), and 50 Kg of Soitem 999/I® (calcium dodecylbenzene sulfonate) are added in a 1000-Liter mixer and stirred until dissolution. 200 gr. of Silicex 140® anti-foaming agent are added. When the mixture is homogenous, 645 Kg of zinc oxide are added little by little and in blocks of 25 Kg, waiting until the product has been assimilated by the system to add the next block.

Once the entire amount of zinc oxide has been added, it is maintained under stirring for 30 minutes to ensure that all the product homogenizes, and the mixture is passed through a vertical micro-ball mill to reduce the particle size to 10 microns, such that there is no more than 0.01 % of particles with a size exceeding 20 microns.

Once all the product has passed through the micro-ball mill, it is left to sit for 2 hours so that it loses the entrained air. A 1% Rhodopol® solution (about 20 Kg) is finally added until obtaining a suspension with a suitable viscosity for the formula, it is adjusted with the amount of water necessary to obtain a suitable concentration, and packaged.
Density: 1.54; pH at 2%: 7.5; Appearance: White-colored viscous liquid.

### Example 2: Suspension with 50% w/v of zinc metal with other surfactants. Formula II.

800 Liters of water, 25 Kg of Descofix 905® (ethoxylated ester of phosphoric acid), and 35 Kg of Nansa 70® / EVM (calcium dodecylbenzene sulfonate) are added in a 1000 Liter mixer and is stirred until dissolution. 200 gr of Silicex 140® anti-foaming agent are added. When the mixture is homogenous, 645 Kg of zinc oxide are added little by little in blocks of 25 Kg, waiting until the product has been assimilated by the system to add the following block.

Once the entire amount of zinc oxide has been added, it is maintained under stirring for 30 minutes to ensure that all the product homogenizes, and the mixture is passed through a vertical micro-ball mill to reduce the particle size to 10 microns, such that there is no more than 0.01 % of particles with a size exceeding 20 microns.

Once all the product has passed through the micro-ball mill, it is left to sit for 2 hours so that it loses the entrained air. A 1% Rhodopol® solution (about 20 Kg) is finally added until obtaining a suspension with a suitable viscosity for the formula, it is adjusted with the amount of water necessary to obtain a suitable concentration, and packaged.
Density: 1.55; pH at 2%: 8.4; Appearance: White-colored viscous liquid.

### Example 3: Suspension with 60% w/v of zinc metal

700 liters of water, 40 Kg of Soitem 132 SP® (tristirylphenol ethoxylate), 80 Kg of Soitem 999/I® (calcium dodecylbenzene sulfonate) are added in a 1000 Liter mixer and stirred until dissolution. 200 gr. of Silicex 140® anti-foaming agent are added. When the mixture is homogenous, 830 Kg of zinc oxide are added little by little and in blocks of 25 Kg, waiting until the product has been assimilated by the system to add the next block.

Once the entire amount of zinc oxide has been added, it is maintained under stirring for 30 minutes to ensure that all the product homogenizes, and the mixture is passed through a vertical micro-ball mill to reduce the particle size to 10 microns, such that there is no more than 0.01 % of particles with a size exceeding 20 microns.

Once all the product has passed through the micro-ball mill, it is left to sit for 2 hours so that it loses the entrained air. A 1% Rhodopol® solution (about 20 Kg) is finally added until obtaining a suspension with a suitable viscosity for the formula, it is adjusted with the amount of water necessary to obtain a suitable concentration, and packaged.
Density: 1.75; pH at 2%: 7.8; Appearance: White-colored viscous liquid.

### Example 4: Suspension with 30% w/v of zinc metal

900 liters of water, 10 Kg of Soitem 132 SP® (tristirylphenol ethoxylate), 20 Kg of Soitem 999/I® (calcium dodecylbenzene sulfonate) are added in a 1000 Liter mixer and stirred until dissolution. 200 gr. of Silicex 140® anti-foaming agent are added. When the mixture is homogenous, 390 Kg of zinc oxide are added little by little and in blocks of 25 Kg, waiting until the product has been assimilated by the system to add the next block.

Once the entire amount of zinc oxide has been added, it is maintained under stirring for 30 minutes to ensure that all the product homogenizes, and the mixture is passed through a vertical micro-ball mill to reduce the particle size to 10 microns, such that there is no more than 0.01 % of particles with a size exceeding 20 microns.

Once all the product has passed through the micro-ball mill, it is left to sit for 2 hours so that it loses the entrained air. A 1% Rhodopol® solution (about 20 Kg) is finally added until obtaining a suspension with a suitable viscosity for the formula, it is adjusted with the amount of water necessary to obtain a suitable concentration, and packaged.
Density: 1.32; pH at 2%: 7.2; Appearance: White-colored viscous liquid.

The products obtained in Examples 1 to 4 have been tested in the control of diarrhea in suckling pigs of between 14 days and 42 days of age, at doses of between 200 and 3000 ppm of zinc oxide. The product was administered both in the drinking water, obtaining acceptance thereof of 100%, and in food. A 20% weight gain was obtained in comparison to those suckling pigs not treated, and an average 85% improvement of the incidence of diarrhea was also obtained.

The level of food consumption improved in all cases since the administration for this formulation did not modify the organoleptic properties of flavor and odor of the food or water.

## Claims

1. A zinc oxide based formulation for preventing and treating diarrhea in farm animals, **characterized in that** it is in liquid form and comprises:
from 30 to 60% by weight of zinc oxide, with a mean particle size of less than 20 microns,
from 1 to 6% of a nonionic surfactant,
from 2.5% to 10% of an ionic surfactant, and
water up to 100% of the volume, all the percentages being by weight.

2. A formulation according to claim 1, **characterized in that** the nonionic surfactant is a tristirylphenol ethoxylate or an ethoxylated ester of phosphoric acid.

3. A formulation according to any of claims 1 and 2, **characterized in that** the ionic surfactant is calcium dodecylbenzene sulfonate or calcium phenyl sulfonate.

4. A formulation according to any of claims 1 to 3, **characterized in that** it additionally comprises one or more thickening agents, flavoring agents, flavor enhancers, proteins, vitamins, antibiotics and other drugs.

5. A formulation according to any of claims 1 to 3, **characterized in that** the farm animals are pigs or rabbits.

6. The use of a liquid formulation according to claims 1 to 5 in the preparation of a medicament for preventing and treating diarrhea in farm animals.

7. The use according to claim 6, **characterized in that** the farm animals are pigs or rabbits.

## Patentansprüche

1. Eine Formulierung auf Zinkoxidbasis zum Vorbeugen und Behandeln von Durchfall bei Nutztieren, **dadurch gekennzeichnet, dass** sie in flüssiger Form ist und Folgendes umfasst:
30 bis 60 Gewichtsprozent Zinkoxid mit einer mittleren Partikelgröße von weniger als 20 Mikrometer;
1 bis 6 % eines nichtionischen Tensids; und
2,5 % bis 10 % eines ionischen Tensids; und
Wasser bis zu 100 % des Volumens, wobei alle Prozentsätze Gewichtsprozente sind.

2. Eine Formulierung nach Anspruch 1, **dadurch gekennzeichnet, dass** es sich bei dem nichtionischen Tensid um ein Tristyrylphenol-Ethoxylat oder einen ethoxylierten Ester von Phosphorsäure handelt.

3. Eine Formulierung nach einem der Ansprüche 1 und 2, **dadurch gekennzeichnet, dass** es sich bei dem ionischen Tensid um Kalzium-DodecylbenzolSulfonat oder Kalzium-Phenyl-Sulfonat handelt.

4. Eine Formulierung nach einem der Ansprüche 1 bis 3, **dadurch gekennzeichnet, dass** sie zusätzlich ein oder mehrere Verdickungsmittel, Geschmacksstoffe, Geschmacksverstärker, Proteine, Vitamine, Antibiotika und andere Arzneimittel umfasst.

5. Eine Formulierung nach einem der Ansprüche 1 bis 3, **dadurch gekennzeichnet, dass** es sich bei den Nutztieren um Schweine oder Kaninchen handelt.

6. Die Verwendung einer flüssigen Formulierung nach Anspruch 1 bis 5 in der Zubereitung eines Medikaments zum Vorbeugen und Behandeln von Durchfall bei Nutztieren.

7. Die Verwendung nach Anspruch 6, **dadurch gekennzeichnet, dass** es sich bei den Nutztieren um Schweine oder Kaninchen handelt.

## Revendications

1. Formulation à base d'oxyde de zinc destinée à prévenir et traiter la diarrhée chez les animaux de la ferme, **caractérisée en ce qu'**elle est sous forme liquide et comprend :
de 30 à 60 % en poids d'oxyde de zinc, avec une taille moyenne des particules inférieure à 20 microns,
de 1 à 6 % d'un agent tensioactif non ionique,
de 2,5 % à 10 % d'un agent tensioactif ionique, et
de l'eau jusqu'à 100 % du volume, tous les pourcentages étant en poids.

2. Formulation selon la revendication 1, **caractérisée en ce que** l'agent tensioactif non ionique est un tristirylphénol éthoxylé ou un ester éthoxylé de l'acide phosphorique.

3. Formulation selon l'une quelconque des revendications 1 et 2, **caractérisée en ce que** l'agent tensioactif ionique est le dodécylbenzènesulfonate de calcium ou le phénylsulfonate de calcium.

4. Formulation selon l'une quelconque des revendications 1 à 3, **caractérisée en ce qu'**elle comprend en outre un ou plusieurs agents d'épaississement, aromatisants, renforçateurs de goût, protéines, vitamines, antibiotiques et autres médicaments.

5. Formulation selon l'une quelconque des revendications 1 à 3, **caractérisée en ce que** les animaux de la ferme sont des porcs ou des lapins.

6. Utilisation d'une formulation liquide selon les revendications 1 à 5 dans la préparation d'un médicament destiné à prévenir et traiter la diarrhée chez les animaux de la ferme.

7. Utilisation selon la revendication 6, **caractérisée en ce que** les animaux de la ferme sont des porcs ou des lapins.
